Europäisches Patentamt
European Patent Office
Office européen des brevets

(19) (11) Veröffentlichungsnummer: **0 301 370 A1**

# (12) EUROPÄISCHE PATENTANMELDUNG

(21) Anmeldenummer: **88111570.3**

(22) Anmeldetag: **19.07.88**

(51) Int. Cl.⁴: **C07D 249/08 , C07D 233/60 , A01N 43/653 , A01N 43/50**

(30) Priorität: **25.07.87 DE 3724645**

(43) Veröffentlichungstag der Anmeldung: **01.02.89 Patentblatt 89/05**

(84) Benannte Vertragsstaaten: **AT BE CH DE ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Schulz, Guenter, Dr.**
**Carl-Bosch-Strasse 96**
**D-6700 Ludwigshafen(DE)**
Erfinder: **Sauter, Hubert, Dr.**
**Neckarpromenade 20**
**D-6800 Mannheim 1(DE)**
Erfinder: **Ammermann, Eberhard, Dr.**
**Sachsenstrasse 3**
**D-6700 Ludwigshafen(DE)**
Erfinder: **Pommer, Ernst-Heinrich, Dr.**
**Berliner Platz 7**
**D-6703 Limburgerhof(DE)**

## (54) Hydroxyethyl-azol-Derivate und ihre Verwendung als Fungizide.

(57) Hydroxyethyl-azol-Derivate der allgemeinen Formel (I),

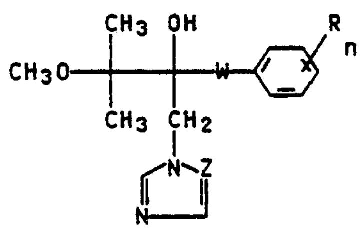

(I)

in welcher
Z für CH oder für N steht,
W für $CH_2$-$CH_2$ oder für CH = CH steht,
R für Wasserstoff, Fluor, Chlor, Brom, Phenyl oder Phenoxy, Benzyl oder Benzyloxy, Alkyl, Halogenalkyl, Thiomethyl, Methoxy, Cyano, Nitro oder Alkoxycarbonyl steht,
n die ganzen Zahlen 1, 2 oder 3 bedeutet,
und deren Säureadditions-Salze und Metallsalz-Komplexe und diese enthaltende Fungizide.

EP 0 301 370 A1

**Hydroxyethyl-azol-Derivate und ihre Verwendung als Fungizide**

Die vorliegende Erfindung betrifft neue Hydroxyethyl-azol-Derivate, Verfahren zu ihrer Herstellung und ihre Verwendung als Fungizide.

Es ist bekannt, daß Hydroxyethyl-azol-Derivate, beispielsweise das 1-(Phenoxy)-3,3-dimethyl-2-(1,2,4-triazol-1-yl-methyl)-2-butanol fungizide Eigenschaften aufweisen (EP 40 345). Die Wirksamkeit dieser Verbindungen ist jedoch insbesondere bei niedrigen Aufwandmengen und -konzentrationen nicht immer ganz zufriedenstellend.

Es wurden nun neue Hydroxyethyl-azol-Derivate der allgemeinen Formel (I),

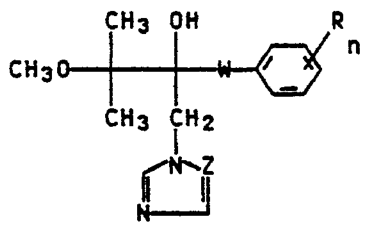

(I)

in welcher
Z für CH oder für N steht,
W für $CH_2$-$CH_2$ oder für CH = CH steht,
R für Wasserstoff, Fluor, Chlor, Brom, Phenyl oder Phenoxy, Benzyl oder Benzyloxy, Alkyl mit bis zu 6 Kohlenstoffatomen, Halogenalkyl mit bis zu 3 Kohlenstoffatomen und bis zu 5 Halogenatomen, Thiomethyl, Methoxy, Cyano, Nitro oder Alkoxycarbonyl mit bis zu 5 Kohlenstoffatomen steht,
n die ganzen Zahlen 1, 2 oder 3 bedeutet, wobei die Substituenten gleich oder verschieden sein können, wenn n größer als 1 ist,
und deren Säureadditions-Salze und Metallsalz-Komplexe gefunden, die eine gute fungizide Wirkung haben.

Weiterhin wurde gefunden, daß man die Hydroxyethyl-azol-Derivate der Formel (I) erhält, wenn man Oxirane der Formel (II),

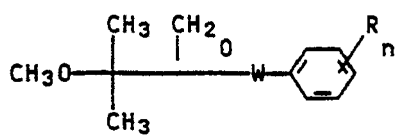

(II)

in der W, R und n die oben angegebene Bedeutung haben, mit Triazol oder Imidazol umsetzt.

Die Umsetzung kann ohne Verdünnungsmittel oder in Gegenwart eines Verdünnungsmittels und ggf. in Gegenwart einer Base durchgeführt werden. Der Ablauf der Reaktion wird schematisch durch die folgende Gleichung 1 (Gl. 1) beschrieben.

(Gl. 1)

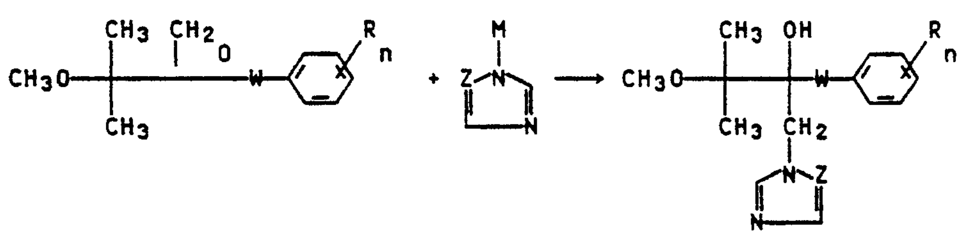

(II) (I)

M bedeutet bedeutet dabei Wasserstoff, wenn ohne Base gearbeitet wird, und z.B. ein Alkalimetall wie Na, Li oder K, wenn in Gegenwart entsprechender alkalimetallhaltiger Basen gearbeitet wird.

An die so erhaltenen Verbindungen der Formel (I) können gegebenenfalls anschließend eine Säure oder ein Metallsalz addiert werden.

Die neuen Hydroxyethyl-azol-Dervate der Formel (I) weisen starke fungizide Eigenschaften auf. Dabei zeigen überraschenderweise die erfindungsgemäßen Verbindungen eine höhere Wirkung als aus dem Stand der Technik bekannte Hydroxyethyl-azol-Derivate wie z. B. 1-(Phenoxy)-3,3-dimethyl-2-(1,2,4-triazol-1-yl-methyl)-2-butanol, welche eine konstitutionell und wirkungsmäßig ähnliche Verbindung ist. Die erfindungsgemäßen Stoffe stellen somit eine Bereicherung der Technik dar.

Die erfindungsgemäßen Hydroxyethyl-azol-Derivate sind durch die Formel (I) allgemein definiert. In dieser Formel stehen vorzugsweise

R für Wasserstoff, Halogenatome - wie Fluor, Chlor oder Brom-, geradkettiges oder verzweigtes Alkyl mit bis zu 6 Kohlenstoffatomen - besonders Methyl, Ethyl, n-Propyl, i-Propyl, n-Butyl, tert.-Butyl, n-Pentyl und n-Hexyl - Halogenalkyl mit bis zu 3 Kohlenstoffatomen und bis zu 5 Halogenatomen - besonders Difluormethyl, Trifluormethyl, Tetrafluorethyl und Pentafluorethyl - Thiomethyl, Methoxy, Cyano, Nitro oder Alkoxycarbonyl mit bis zu 5-Kohlenstoffatomen - besonders Methoxycarbonyl, Ethoxycarbonyl, Propoxycarbonyl und tert.-Butoxycarbonyl -

W für $-CH_2CH_2-$ oder $-CH=CH-$

Z für CH oder für N

n für 1, 2 oder 3 - besonders für 1 und 2 - wobei die Substituenten gleich oder verschieden sein können, wenn n größer als 1 ist.

Erfindungsgemäße Verbindungen sind auch Additionsprodukte aus Säuren und Hydroxyethyl-azol-Derivaten der Formel (I).

Zu den Säuren, die addiert werden können, gehören z.B. Halogenwasserstoffsäuren, z.B. Chlorwasserstoffsäure oder Bromwasserstoffsäure, insbesondere die Chlorwasserstoffsäure, ferner Phosphorsäure, Salpetersäure, Carbonsäuren, z.B. Essigsäure, Maleinsäure, Bernsteinsäure, Weinsäure, Zitronensäure, Salicylsäure oder Milchsäure, sowie Sulfonsäuren, z.B. Dodecyclbenzolsulfonsäure, p-Toluolsulfonsäure und Naphthalinsulfonsäure.

Erfindungsgemäße Verbindungen sind auch Additionsprodukte von Salzen von Metallen der II. bis IV. Haupt- und der I. und II. sowie IV. bis VIII. Nebengruppe des Periodensystems und substituierten Hydroxyethyl-azol-Derivaten der Formel (I).

Hierbei sind Salze des Kupfers, Zinks, Mangans, Magnesiums, Zinns, Eisen und Nickels besonders bevorzugt. Als Anionen der Salze kommen solche in Betracht, die sich von Säuren ableiten, die zu physiologisch verträglichen Additionsprodukten führen. Besonders bevorzugte derartige Säuren sind in diesem Zusammenhang die Halogenwasserstoffsäuren wie z. B. die Chlorwasserstoffsäure und die Bromwasserstoffsäure, ferner Phosphorsäure, Salpetersäure und Schwefelsäure. Salze sind z.B. Kupfersulfat, Zinkchlorid, Zinksulfat, Mangansulfat, Magnesiumchlorid, Zinnchlorid, Eisensulfat, Nickelsulfat. Kupfersulfat wird bevorzugt.

Die Durchführung des Verfahrens zur Herstellung der Hydroxyethyl-azol-Derivate der Formel (I) aus den Oxiranen der Formel (II) erfolgt nach dem Schema, das in Gleichung 1 (Gl. 1) angegeben ist. Dabei setzt man entweder Triazol oder Imidazol im stöchiometrischen Verhältnis oder im Überschuß in Substanz oder in Gegenwart eines inerten organischen Lösungsmittels -besonders sei hier auf N-Methylpyrrolidon hingewiesen - bei Temperaturen zwischen 60 und 150° C um. Zur Unterstützung der Umsetzung können Basen wie LiOH, NaOH oder KOH zugesetzt werden oder man überführt Triazol oder Imidazol separat in ihr Alkalimetallsalz und setzt dieses in die Reaktion ein.

Die Oxirane der Formel (II) können auf allgemein bekannte Art und Weise hergestellt werden, indem man von entsprechenden Ketonen ausgeht. Als Reagenzien zur Überführung der Ketofunktion in den Oxiran-Ring sind z. B. Dimethyloxosulfoniummethylid oder Dimethylsulfoniummethylid geeignet. Diese Verfahren sind in der Literatur bekannt (vgl. z. B. J. Am. Chem. Soc. 87, 1363 - 1364 (1965) oder Heterocycles 8, 397 (1977)) und in Gleichung 2a und 2b (Gl. 2a, Gl. 2b) schematisch dargestellt.

Gl. 2a 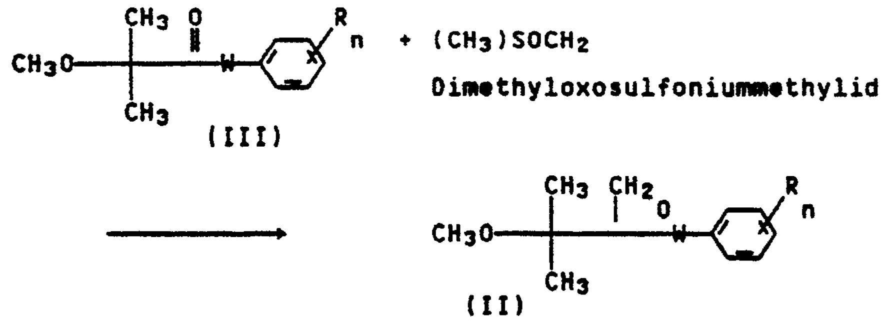


(III) (II)

Gl. 2b 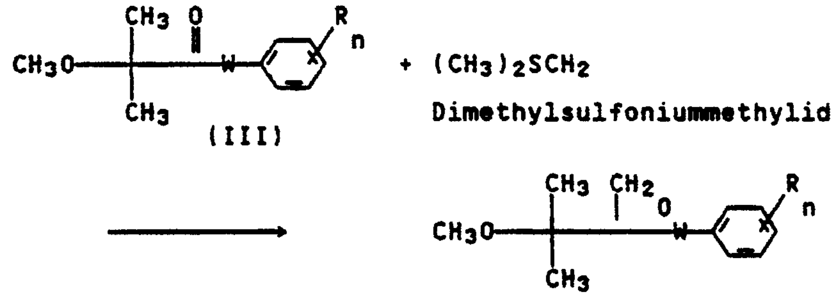


(III) (II)

Ketone der Formel (III),

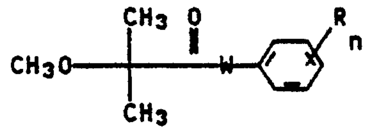

in der W und R sowie n die vorher angegebene Bedeutung haben, erhält man durch Kondensationsreaktion von 3-Methoxy-3-methylbutanon-(2) mit aromatischen Aldehyden der Formel (IV) und anschließender Hydrierung, wenn W = $CH_2$-$CH_2$ ist, oder ohne Hydrierung, wenn W = -CH=CH- ist, was schematisch in Gleichung 3 (Gl. 3) und Gleichung 4 (Gl. 4) gezeigt ist.

Gl. 3 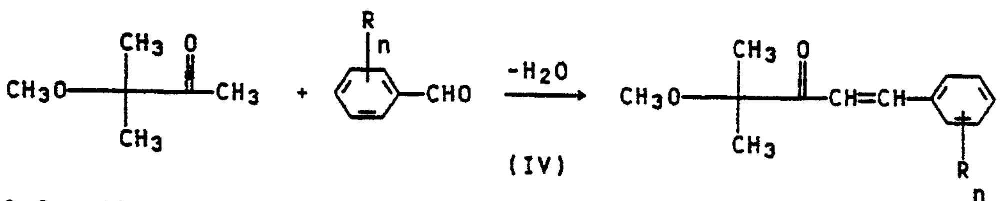


(IV)

3-Methyl-3-methoxybutanon-(2)

Gl. 4 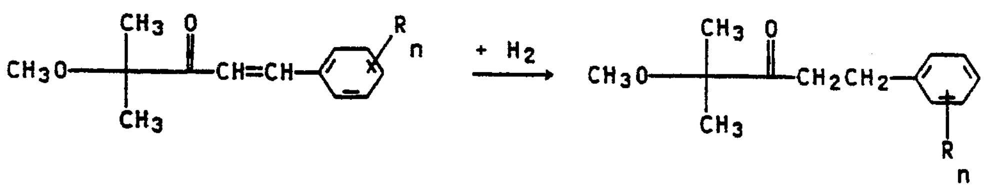


Der Substituent R und die Zahl n in den aromatischen Aldehyden der Formel (IV). haben die vorher angegebene Bedeutung. Die Durchführung der Kondensation erfolgt unter den allgemein üblichen Bedingungen, zum Beispiel durch den katalytischen Einfluß von Basen wie KOH und Auskreisen von Wasser. Die Hydrierung der Kondensationsprodukte entsprechend Gleichung 4 kann katalytisch mit Wasserstoff bei Normaldruck oder erhöhten Druck durchgeführt werden. Die Reaktion kann damit in allgemein üblicher Weise erfolgen. Als Katalysatoren sind z.B. PdO auf $Al_2O_3$ geeignet. Als Lösungsmittel können z. B. niedere aliphatische Alkohole wie Methanol oder Ethanol eingesetzt werden.

Zur Illustration der beschriebenen Vorgehensweise und des Verfahrens zur Herstellung von

Hydroxyethyl-azol-Derivaten werden die folgenden Beispiele angeführt.

Vorschrift 1

Herstellung von 4-Methoxy-4-methyl-1-phenyl-pent-1-en-3-on (Vorprodukt, Beispiel zu Gleichung 3).

116 g 3-Methoxy-3-methylbutanon-(2) und 102 ml Benzaldehyd in 500 ml Toluol werden in Gegenwart von 2 g pulverisierter KOH am Wasserabscheider gekocht. Nachdem ca. 15 ml Wasser ausgekreist worden sind, kühlt man ab, filtriert und befreit i. Vak. vom Lösungsmittel: 198 g Öl
$^1$H-NMR ($CDCl_3$):
1,4 s (6H), 3,22 s (3H), 7,35 m (3H), 7,38 d (1H), 7,6 m (2H), 7,78 d (1H).

Vorschrift 2

Herstellung von 2-Methoxy-2-methyl-5-(4-methylphenyl)-pentan-3-on (Vorprodukt, Beispiel zu Gleichung 4).

109 g 4-Methoxy-4-methyl-1-(4-methylphenyl)-penten-1-en-3-on in 500 ml MeOH werden in Gegenwart von 0,5 % PdO auf $Al_2O_3$ und 2 g $Na_2CO_3$ in 10 ml Wasser mit Wasserstoff unter Normaldruck bei 50 - 60 °C behandelt. Wenn kein Wasserstoff mehr aufgenommen wird, isoliert man das Produkt auf die übliche Weise: 107 g Öl
$^1$H-NMR ($CDCl_3$):
1,25 s (6H), 2,3 s (3H), 2,87 m (4H), 3,15 s (3H), 7,1 s (4H).

Vorschrift 3

Herstellung von 2-(1-Methoxy-1-methyl)ethyl-2[2-(4-tert.-butylphenyl)]ethyloxiran (Vorprodukt, Beispiel zu Gleichung 2b).

Zu 13,5 g Kalium-tert.-butylat in 100 ml tert.-Butanol werden 19 g Trimethylsulfoniumiodid und 18,1 g 2-Methoxy-2-methyl-5-(4-tert.-butylphenyl)pentan-3-on gegeben. Man hält bei 60 - 70 °C bis kein Keton mehr im Dünnschichtchromatogramm (DC) nachweisbar ist. Isolieren des Oxirans in üblicher Weise liefert 16,5 g Öl.
($CDCl_3$):
1,2 s (3H), 1,25 s (3H); 1,35 s (9H), (2,05-2,3) und (2,4-2,65) jeweils m insgesamt (4H), 2,67 AB-Dublett: 3,3 s (3H), 7,1-7,35 m (4H).

Herstellungsbeispiel

Herstellung von 2-Methoxy-2-methyl-5-(4-methylphenyl)3-(1,2,4-triazol-1-yl-methyl)-pentan-3-ol (Wirkstoff Nr. 69 in der Tabelle)

62,7 g 2-(1-Methoxy-1-methyl)ethyl-2-[2-(4-methylphenyl)]ethyloxiran werden mit 37 g Triazol und 6,4 g LiOH in 500 ml N-Methylpyrrolidon auf 100 -130 °C erwärmt bis nach DC das Oxiran vollständig umgesetzt worden ist. Nach Entfernen des Lösungsmittels und chromatographischer Aufarbeitung erhält man 41,9 g kristallines Produkt. Fp. 67 - 70 °C.

$^1H$-NMR ($CDCl_3$):
1,19 s (3H); 1,21 s (3H); 1,75 m (2H, AB-System); 2,25 m (1H) + 2,62 m (1H) AB-System; 2,35 s (3H), 3,21 s (3H); 3,4 s (1H) breit, 4,42 AB-Quartett (2H); 7,05 m (4H); 7,95 s (1H), 8,2 s (1H).

Entsprechend den Beispielen können die in folgender Tabelle zusammengestellten Wirkstoffe hergestellt werden.

| Nr. | Z | W | $R_n$ | Fp [°C] |
|---|---|---|---|---|
| 1 | N | $CH_2CH_2$ | H | 93 |
| 2 | N | CH=CH | H | 102 |
| 3 | CH | $CH_2CH_2$ | H | |
| 4 | CH | CH=CH | H | |
| 5 | N | $CH_2CH_2$ | 4-Cl | Öl*) |
| 6 | N | CH=CH | 4-Cl | 120 |
| 7 | CH | $CH_2CH_2$ | 4-Cl | |
| 8 | CH | CH=CH | 4-Cl | 191 |
| 9 | N | $CH_2CH_2$ | 2,4-$Cl_2$ | |
| 10 | N | CH=CH | 2,4-$Cl_2$ | 82 |
| 11 | CH | $CH_2CH_2$ | 2,4-$Cl_2$ | |
| 12 | CH | CH=CH | 2,4-$Cl_2$ | 148 |
| 13 | N | $CH_2CH_2$ | 2-Cl | Öl*) |
| 14 | N | CH=CH | 2-Cl | 120 |
| 15 | CH | $CH_2CH_2$ | 2-Cl | |
| 16 | CH | CH=CH | 2-Cl | |
| 17 | N | $CH_2CH_2$ | 3-Cl | Öl*) |
| 18 | N | CH=CH | 3-Cl | 137-140 |
| 19 | CH | $CH_2CH_2$ | 3-Cl | |
| 20 | CH | CH=CH | 3-Cl | 144 |
| 21 | N | $CH_2CH_2$ | 2-F | |
| 22 | N | CH=CH | 2-F | |
| 23 | CH | $CH_2CH_2$ | 2-F | |
| 24 | CH | CH=CH | 2-F | |
| 25 | N | $CH_2CH_2$ | 4-F | |
| 26 | N | CH=CH | 4-F | |
| 27 | CH | $CH_2CH_2$ | 4-F | |
| 28 | CH | CH=CH | 4-F | |
| 29 | N | $CH_2CH_2$ | 3-Br | |
| 30 | N | CH=CH | 3-Br | |
| 31 | CH | $CH_2CH_2$ | 3-Br | |
| 32 | CH | CH=CH | 3-Br | |
| 33 | N | $CH_2CH_2$ | 3-F | |
| 34 | N | CH=CH | 3-F | |
| 35 | N | $CH_2CH_2$ | 3-F | |
| 36 | N | CH=CH | 3-F | |
| 37 | N | $CH_2CH_2$ | 2-F, 6-Cl | |

| Nr. | Z | W | $R_n$ | Fp [°C] |
|---|---|---|---|---|
| 38 | N | CH=CH | 2-F, 6-Cl | |
| 39 | CH | $CH_2CH_2$ | 2-F, 6-Cl | |
| 40 | CH | CH=CH | 2-F, 6-Cl | |
| 41 | N | $CH_2CH_2$ | 4-CN | |
| 42 | N | CH=CH | 4-CN | Öl |
| 43 | CH | $CH_2CH_2$ | 4-CN | |
| 44 | CH | CH=CH | 4-CN | |
| 45 | N | $CH_2CH_2$ | $4-OC_6H_5$ | |
| 46 | N | CH=CH | $4-OC_6H_5$ | |
| 47 | CH | $CH_2CH_2$ | $4-OC_6H_5$ | |
| 48 | CH | CH=CH | $4-OC_6H_5$ | |
| 49 | N | $CH_2CH_2$ | $4-OCF_2CF_2H$ | |
| 50 | N | CH=CH | $4-OCF_2CF_2H$ | |
| 51 | CH | $CH_2CH_2$ | $4-OCF_2CF_2H$ | |
| 52 | CH | CH=CH | $4-OCF_2CF_2H$ | |
| 53 | N | $CH_2CH_2$ | $4-CH(CH_3)_2$ | |
| 54 | N | CH=CH | $4-CH(CH_3)_2$ | 108 |
| 55 | CH | $CH_2CH_2$ | $4-CH(CH_3)_2$ | |
| 56 | CH | CH=CH | $4-CH(CH_3)_2$ | 121 |
| 57 | N | $CH_2CH_2$ | $4-C(CH_3)_3$ | Öl |
| 58 | N | CH=CH | $4-C(CH_3)_3$ | Harz |
| 59 | CH | $CH_2CH_2$ | $4-C(CH_3)_3$ | |
| 60 | CH | CH=CH | $4-C(CH_3)_3$ | |
| 61 | N | $CH_2CH_2$ | $4-C_6H_5$ | |
| 62 | N | CH=CH | $4-C_6H_5$ | 142-145 |
| 63 | CH | $CH_2CH_2$ | $4-C_6H_5$ | |
| 64 | CH | CH=CH | $4-C_6H_5$ | 157 |
| 65 | N | $CH_2CH_2$ | $4-CF_3$ | |
| 66 | N | CH=CH | $4-CF_3$ | |
| 67 | CH | $CH_2CH_2$ | $4-CF_3$ | |
| 68 | CH | CH=CH | $4-CF_3$ | |
| 69 | N | $CH_2CH_2$ | $4-CH_3$ | 67-70 |
| 70 | N | CH=CH | $4-CH_3$ | |
| 71 | CH | $CH_2CH_2$ | $4-CH_3$ | |
| 72 | CH | CH=CH | $4-CH_3$ | 139 |
| 73 | N | $CH_2CH_2$ | $4-OCH_3$ | |
| 74 | N | CH=CH | $4-OCH_3$ | |
| 75 | CH | $CH_2CH_2$ | $4-OCH_3$ | |
| 76 | CH | CH=CH | $4-OCH_3$ | |
| 77 | N | $CH_2CH_2$ | $4-NO_2$ | |
| 78 | N | CH=CH | $4-NO_2$ | |

| Nr. | Z | W | $R_n$ | Fp [°C] |
|---|---|---|---|---|
| 79 | CH | $CH_2CH_2$ | 4-$NO_2$ | |
| 80 | CH | CH=CH | 4-$NO_2$ | |
| 81 | N | $CH_2CH_2$ | 4-$CO_2CH_3$ | |
| 82 | N | CH=CH | 4-$CO_2CH_3$ | |
| 83 | CH | $CH_2CH_2$ | 4-$CO_2CH_3$ | |
| 84 | CH | CH=CH | 4-$CO_2CH_3$ | |
| 85 | N | $CH_2CH_2$ | 4-$CH_2C_6H_5$ | |
| 86 | N | CH=CH | 4-$CH_2C_6H_5$ | |
| 87 | CH | $CH_2CH_2$ | 4-$CH_2C_6H_5$ | |
| 88 | CH | CH=CH | 4-$CH_2C_6H_5$ | |
| 89 | N | $CH_2CH_2$ | 4-$SCH_3$ | |

*) durch $^1$H-NMR charakterisiert:

Nr. 5:
1,19 s 3H; 1,21 s 3H; 1,78 m 2H; (2,28+2,62) AB-Typ Multiplett 2H; 3,2 s 3H; 4,45 AB Multiplett 2H; 6,97-7,40 m 4H; 7,95 s 1H; 8,20 s 1H.

Nr. 13:
1,25 s 3H; 1,28 s 3H; 1,7 m 2H; (2,5+2,8) AB-Typ Multiplett 2H; 3,2 s 3H, 4,45 AB Multiplett 2H; 7,0-7,35 m 4H 7,95 s 1H; 8,25 s 1H.

Nr. 17:
1,20 s 3H; 1,21 3H; 1,70 m 2H; (2,22+2,65) AB-Typ Multiplett 2H; 3,25 s 3H, 4,42 AB Multiplett 2H; 6,9-7,3 m 4H; 7,95 s 1H; 8,21 s 1H.

Nr. 42:
1,25 s 3H; 1,30 s 3H; 3,3 s 3H; 3,95 s breit 14; (4,45 d 1H + 4,61 d 1H) AB-System; (6,35 d 1 H + 6,48 d 1H) AB-System; 7,30 m 2H; 7,52 m 2H; 7,83 s 1H; 8,09 s 1H.

Nr. 57:
1,20 s 3H; 1,22 s 3H; 1,30 s 9H; 1,79 m 2H; (2,28+2,62) AB-Typ Multiplett 2H; 3,2 s 3H; 4,42 AB Multiplett 2H; 7,03 m 2H; 7,38 m 2H; 7,91 s 1H; 8,20 s 1H.

Nr. 58:
1,20 s 3H; 1,28 s 3H; 1,29 s 9H; 3,28 s 3H; 3,65 s breit 1H; (4,43 d 1H + 4,58 d 1H) AB-System; (6,19 d 1H + 6,45 d 1H) AB-System; 7,1-7,35 m 4H; 7,8 s 1H; 8,1 s 1H.

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Einige der neuen Verbindungen haben sehr gute Wirksamkeit gegen humanpathogene Pilze, wie Trichophyton mentagrophytes und Candida albicans.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-$\alpha$-pyrrolidon und erhält eine Lösung, die zur Anwendung in Form kleinster Tropfen geeignet ist.

II. 20 Gew.-Teile der Verbindung Nr. 5 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 13 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 17 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 1 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 5 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 13 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 17 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 1 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N′-propylen-bis-dithiocarbamat),
Zink-(N,N′-propylen-bis-dithiocarbamat),
N,N′-Polypropylen-bis-(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester

heterocyclische Substanzen, wie
2-Heptadecyl-2-imidazolin-acetat,
2,4-Dichlor-6-(o-chloranilino)-s-triazin,
0,0-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,

N-Dichlorfluormethylthio-N′,N′-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N′-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
$\alpha$-(2-Chlorphenyl)-$\alpha$-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2′-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,

3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3--chlor-2-aminopyridin,
1-((bis-(4-Fluorphenyl)-methylsilyl)-methyl)-1H-1,2,4-triazol.

## Anwendungsbeispiele

Als Vergleichswirkstoff wurde 1-Phenoxy-3,3-dimethyl-2-(1,2,4-triazolyl-1-methyl)-2-butanol (A) - bekannt aus EP 40 345 - benutzt.

## Anwendungsbeispiel 1

### Wirksamkeit gegen Pyrenophora teres

Gerstenkeimlinge der Sorte "Asse" wurden im Zweiblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18 °C gestellt. Anschließend wurden die Pflanzen im Gewächshaus bei 20 bis 22 °C und 70 % relativer Luftfeuchtigkeit für weitere 5 Tage kultiviert. Dann wurde das Ausmaß der Symptomentwicklung ermittelt.

Das Ergebnis zeigt, daß die Wirkstoffe 1, 5, 13, 17 und 57 bei der Anwendung als 0,05 %ige (Gew.%) Spritzbrühe eine besere fungizide Wirkung zeigen (100 %) als der bekannte Vergleichswirkstoff A (70 %).

## Anwendungsbeispiel 2

### Wirksamkeit gegen Botrytis cinerea an Paprika

Paprikasämlinge der Sorte "Neusiedler Ideal Elite" wurden, nachdem sich 4-5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22 bis 24 °C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten.

Das Ergebnis zeigt, daß die Wirkstoffe 5 und 17 bei der Anwendung als 0,05 %ige Spritzbrühe eine bessere fungizide Wirkung zeigen (97 %) als der bekannte Vergleichswirkstoff A (0 %).

## Ansprüche

1. Hydroxyethyl-azol-Derivate der allgemeinen Formel (I),

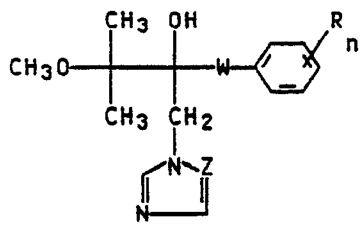

(I)

in welcher
Z für CH oder für N steht,
W für $CH_2$-$CH_2$ oder für CH = CH steht,
R für Wasserstoff, Fluor, Chlor, Brom, Phenyl oder Phenoxy, Benzyl oder Benzyloxy, Alkyl mit bis zu 6 Kohlenstoffatomen, Halogenalkyl mit bis zu 3 Kohlenstoffatomen und bis zu 5 Halogenatomen, Thiomethyl, Methoxy, Cyano, Nitro oder Alkoxycarbonyl mit bis zu 5 Kohlenstoffatomen steht,
n die ganzen Zahlen 1, 2 oder 3 bedeutet, wobei die Substituenten gleich oder verschieden sein können, wenn n größer als 1 ist, und deren Säureadditions-Salze und Metallsalz-Komplexe.

2. Verfahren zur Herstellung von Verbindungen der Formel I gemäß Anspruch 1 gekennzeichnet dadurch, daß man Triazol oder Imidazol mit Oxiranen der Formel (II),

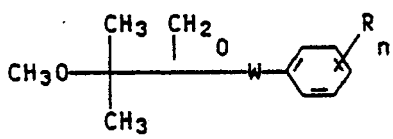

(II)

in der W, R und n die in Anspruch 1 angegebene Bedeutung haben, umsetzt.

3. Fungizides Mittel, enthaltend ein Hydroxyethyl-azol-Derivat der allgemeinen Formel I

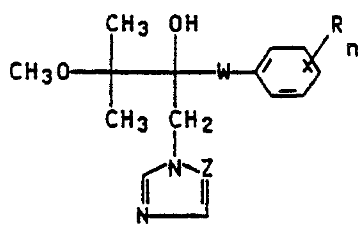

(I)

in welcher
Z für CH oder für N steht,
W für $CH_2$-$CH_2$ oder für CH = CH steht,
R für Wasserstoff, Fluor, Chlor, Brom, Phenyl oder Phenoxy, Benzyl oder Benzyloxy, Alkyl mit bis zu 6 Kohlenstoffatomen, Halogenalkyl mit bis zu 3 Kohlenstoffatomen und bis zu 5 Halogenatomen, Thiomethyl, Methoxy, Cyano, Nitro oder Alkoxycarbonyl mit bis zu 5 Kohlenstoffatomen steht,
n die ganzen Zahlen 1, 2 oder 3 bedeutet, wobei die Substituenten gleich oder verschieden sein können, wenn n größer als 1 ist, oder dessen Säureadditions-Salz oder Metallsalz-Komplex und einen festen oder flüssigen Trägerstoff.

4. Verfahren zur Herstellung von Fungiziden, gekennzeichnet dadurch, daß man eine Verbindung der Formel I gemäß Anspruch 1 mit einem festen oder flüssigen Trägerstoff mischt.

5. Verfahren zur Bekämpfung von Fungi, gekennzeichnet dadurch, daß man ein Hydroxyethylazolderivat der allgemeinen Formel I

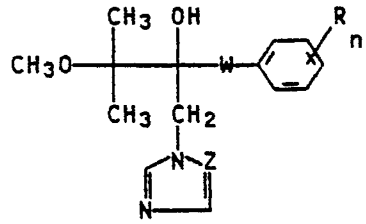

(I)

in welcher
Z für CH oder für N steht,
W für $CH_2$-$CH_2$ oder für CH = CH steht,
R für Wasserstoff, Fluor, Chlor, Brom, Phenyl oder Phenoxy, Benzyl oder Benzyloxy, Alkyl mit bis zu 6 Kohlenstoffatomen, Halogenalkyl mit bis zu 3 Kohlenstoffatomen und bis zu 5 Halogenatomen, Thiomethyl, Methoxy, Cyano, Nitro oder Alkoxycarbonyl mit bis zu 5 Kohlenstoffatomen steht,
n die ganzen Zahlen 1, 2 oder 3 bedeutet, wobei die Substituenten gleich oder verschieden sein können, wenn n größer als 1 ist, oder dessen Säureadditions-Salz oder Metallsalz-Komplex auf Pilze oder durch Pilzbefall bedrohte Substanzen, Pflanzen oder Saatgüter einwirken läßt.

6. Hydroxylethyl-azol-derivat gemäß Anspruch 1, dadurch gekennzeichnet, daß Z N, W $CH_2$-$CH_2$ und R Wasserstoff, Chlor oder $C_4$-Alkyl bedeutet.

7. 2-Methoxy-2-methyl-5-(4-chlorphenyl)-3-(1,2,4-triazol-1-yl-methyl)-pentan-3-ol.

8. 2-Methoxy-2-methyl-5-phenyl-3-(1,2,4-triazol-1-yl-methyl)-pentan-3-ol.

9. 2-Methoxy-2-methyl-5-(2-chlorphenyl)-3-(1,2,4-triazol-1-yl-methyl)-pentan-3-ol.

Europäisches Patentamt

# EUROPÄISCHER RECHERCHENBERICHT

Nummer der Anmeldung

EP 88 11 1570

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 110 048 (BAYER AG)<br>* Seiten 1-7 * | 1-9 | C 07 D 249/08<br>C 07 D 233/60<br>A 01 N 43/653<br>A 01 N 43/50 |
| Y | EP-A-0 061 835 (ICI)<br>* Seite 1 * | 1-9 | |
| Y,D | EP-A-0 040 345 (BAYER AG)<br>* Beispiele * | 1-9 | |
| A | DE-A-3 315 808 (BAYER AG)<br>* Seiten 25-27 * | 1 | |
| A | DE-A-3 242 249 (BAYER AG)<br>* Seiten 1-12 * | 1 | |
| | | | **RECHERCHIERTE SACHGEBIETE (Int. Cl.4)** |
| | | | C 07 D 249/00<br>C 07 D 233/00<br>A 01 N 43/00 |

Der vorliegende Recherchenbericht wurde für alle Patentansprüche erstellt

| Recherchenort | Abschlußdatum der Recherche | Prüfer |
|---|---|---|
| DEN HAAG | 16-11-1988 | BRIGHENTI |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)